(19) Europäisches Patentamt
European Patent Office
Office européen des brevets

(11) Numéro de publication: **0 027 412**
**B1**

# (12) FASCICULE DE BREVET EUROPEEN

(45) Date de publication du fascicule du brevet: **14.12.83**

(21) Numéro de dépôt: **80401433.0**

(22) Date de dépôt: **08.10.80**

(51) Int. Cl.³: **A 61 K 31/44,**
A 61 K 31/33,
C 07 D 213/56,
C 07 D 213/57,
C 07 D 401/06,
C 07 D 413/06

## (54) Dérivés de la pyridine, procédé pour leur préparation et médicaments les contenant.

(30) Priorité: **11.10.79 FR 7925370**

(43) Date de publication de la demande:
**22.04.81 Bulletin 81/16**

(45) Mention de la délivrance du brevet:
**14.12.83 Bulletin 83/50**

(84) Etats contractants désignés:
**AT BE CH DE FR GB IT LI LU NL SE**

(56) Documents cités:
**FR - M - 643**
**FR - M - 2 485**
**GB - A - 666 778**

(73) Titulaire: **SANOFI, société anonyme**
**40, Avenue George V**
**F-75008 Paris (FR)**

(72) Inventeur: **Demarne, Henri**
**Le Florence 65 avenue du Major Flandre**
**F-34 000 Montpellier (FR)**
Inventeur: **Bernhart, Claude**
**144 rue des Muriers**
**F-34980 Saint Gely du Fesc (FR)**
Inventeur: **Lansen, Jacqueline**
**27 allée de Bon Accueil**
**F-34100 Montpellier (FR)**

(74) Mandataire: **Combe, André et al,**
**CABINET BEAU DE LOMENIE 55 rue d'Amsterdam**
**F-75008 Paris (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 027 412 B1

Dérivés de la pyridine, procédé pour leur préparation et médicaments les contenant

La présente invention concerne en tant que produits industriels nouveaux des dérivés de la pyridine, ainsi que leurs méthodes de préparation et leur application en thérapeutique.

Le BSM 2485 M décrit des amines $\alpha$-arylés analogues aux composés de l'invention et possédant de bonnes qualités cardio-régulatrices.

Les nouveau composés de l'invention répondent à la formule générale:

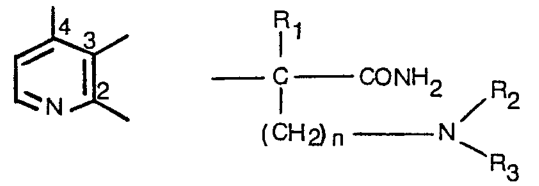

A B (I)

dans laquelle:

—le groupe B substitue la pyridine en position 2, 3, ou 4;

—$R_1$ représente un groupe alkyle droit ou ramifié saturé ou insaturé ayant 1 à 10 atomes de carbone, un groupe cyclohexyle ou cyclohexylméthyle

—$R_2$ et $R_3$ représentent un groupe alkyle droit ou ramifié ayant 1 à 6 atomes de carbone ou un groupe cyclohexyle ou encore les 2 groupes $R_2$ et $R_3$ avec l'atome d'azote auquel ils sont liés constituent un hétérocycle choisi parmi le groupe morpholino et le dimethyl-2,6 pipéridino.

—n étant égal à 2, 3 ou 4.

Les composés (I) fournissent avec les acides organiques ou minéraux des sels solubles. Ces sels avec les acides pharmaceutiquement acceptables font partie intégrante de l'invention.

Les composés selon l'invention sont obtenus selon l'un ou l'autre des schémas réactionnels suivant : méthode A lorsque $R_1$ désigne un radical saturé, méthode B lorsque $R_1$ désigne un radical insaturé.

Méthode A:

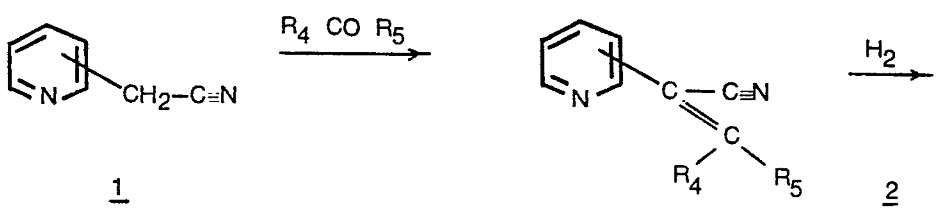


1 2

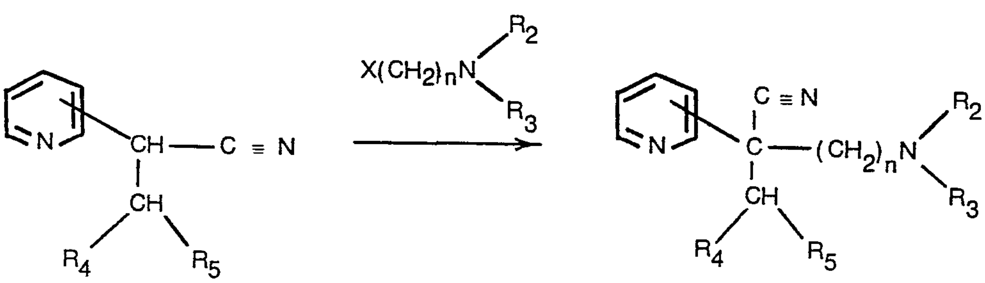

3 4

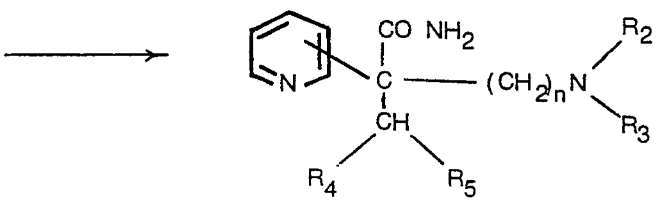

(I)

Le pyridyl acétonitrile *1* traité par un aldéhyde ou une cétone $R_4$ CO $R_5$ ($R_4$ = H ou alkyle, $R_5$ = alkyle) en présence d'acide acétique et de pipéridine conduit au nitrile éthylénique *2*. Celui-ci est

réduit catalytiquement en nitrile saturé *3*. Ce dernier, soumis à une réaction d'alkylation par une chaîne halogénée

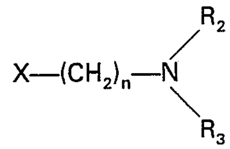

(X = halogène) conduit au composé *4* dont on hydrolyse la fonction nitrile en amide pour aboutir au composé I.

Le choix judicieux des radicaux $R_4$ et $R_5$ de l'aldéhyde ou de la cétone utilisés pour préparer *2* permet, dans le composé final I, d'obtenir des radicaux $R_1$ droits, cycliques ou ramifiés saturés.

Méthode B:

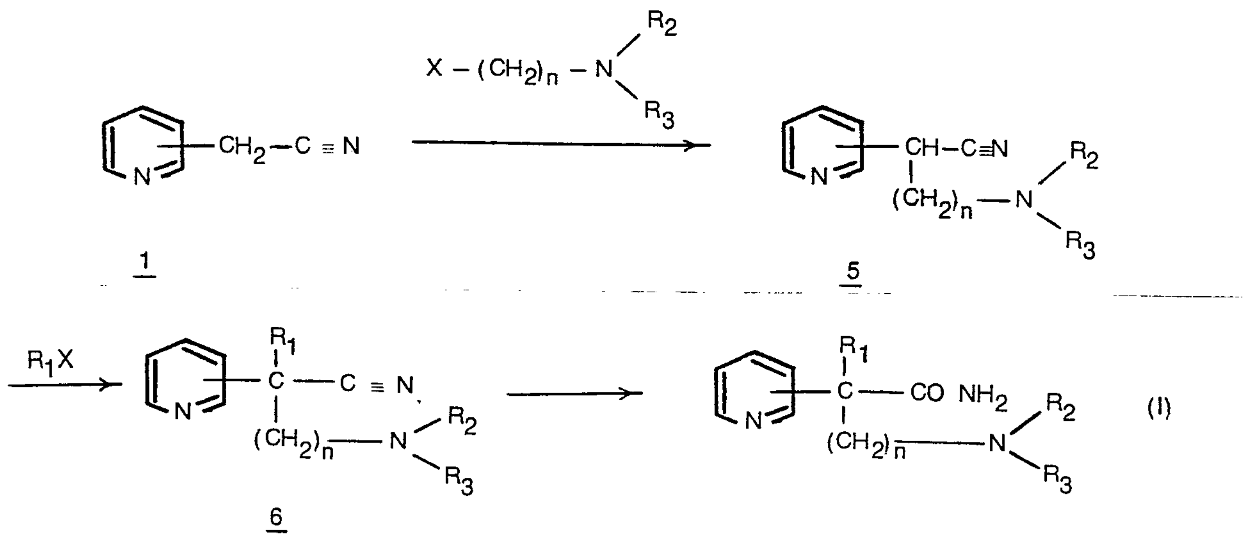


1 5 6 (I)

Le pyridyl acétonitrile *1* d'abord soumis à une réaction d'alkylation en présence d'une chaîne halogénée:

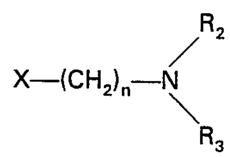

(X = halogène) pour conduire au nitrile 5.

Ce dernier est ensuite soumis à une deuxième réaction d'alkylation en présence d'un composé $R_1X$ (X = halogène), conduisant ainsi au nitrile *6*. On obtient finalement les composés (I) par hydrolyse du nitrile en présence de potasse en milieu hydro-alcoolique.

Les exemples suivants nullement limitatifs sont donnés á titre d'illustration pour le préparation des composés (I) suivant l'une ou l'autre des méthodes A ou B.

Exemple 1 (méthode A)

*(Diisoproylamino-2 éthyl)-2 (pyridyl-2)-2 méthyl-4 pentanamide.*

(CM 7857)

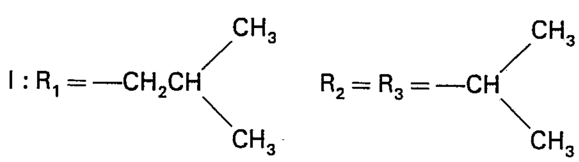

n = 2

a) Méthyl-4 (pyridyl-2)-2 pentène-2 nitrile.

Dans un ballon équipé d'un séparateur d'eau, on place 7 g de pyridyl-2 acétonitrile, 12,8 g d'isobutyraldéhyde, 0,9 ml d'acide acétique, 0,18 ml de pipéridine et 250 ml de benzène sec. On chauffe au reflux pendant 3 heures. Après refroidissement, on lave le mélange réactionnel avec de l'eau, sèche la phase organique sur sulfate de sodium puis évapore le solvant à siccité.

Par distillation du résidu, on obtient un liquide jaune (9,9 g); E./1,4 mm de Hg : 89—92°C.

b) Méthyl-4 (pyridyl-2)-2 pentane nitrile.

On hydrogène à température et pression ambiantes la solution de 9 g du composé obtenu ci-dessus, dissous dans 100 ml d'éthanol à 96° en présence de 3,8 g de charbon palladié à 5%. On filtre le mélange réactionnel et évapore le filtrat à siccité.

On obtient un liquide jaune (9 g) utilisé tel quel pour la suite.

c) (Diisopropylamino-2 éthyl)-2 (pyridyl-2)-2 méthyl-4 pentane nitrile.

On chauffe au reflux pendant 2 heures le mélange de 9 g du nitrile précédent, 9,3 g de chloro-1 dissopropylamino-2 éthane et 2,2 g d'amidure de sodium pulvérisé dans 150 ml de toluène sec. On lave le mélange réactionnel avec de l'eau puis sèche la phase organique sur sulfate de sodium et évapore à siccité.

On obtient un liquide orangé (15,1 g) utilisé tel quel pour l'opération suivante.

d) CM 7857

On chauffe à 120°C pendant 1 heure, 15,1 g du composé obtenu précédemment dans 100 ml d'acide sulfurique d = 1,83. Après refroidissement, on verse la solution en agitant sur 600 g de glace pilée. On alcalinise avec une solution de soude à 40% et on extrait avec du chloroforme. On sèche la phase organique sur sulfate de sodium puis évapore le solvant à siccité. On chromatographie le résidu sur une colonne d'alumine en éluant avec un mélange pentane-acétate d'éthyle.

On obtient un solide incolor (6,9 g).

Après recristallisation dans l'éther isopropylique, F : 107—108°C.

Exemple 2 (méthode B)

*(Diisopropylamino-2 éthyl)-2 (pyridyl-2)-2 méthyl-4 pentène-4 amide.*

(CM 40348

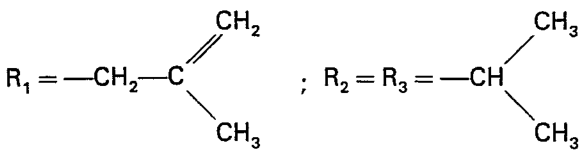

; n = 2

a) Diisopropylamino-4 (pyridyl-2)-2 butanenitrile.

Dans un ballon on place 8 g de pyridyl-2 acétonitrile, 8,81 g de chloro-1 diisopropylamino-2 éthane et 0,27 g de chlorure de benzyl triéthyl ammonium. En maintenant la température en dessous de 35°C, on ajoute 35 $cm^3$ de soude à 50%. Le mélange est chauffé à 35°C pendant 5 heures. Après retour à température ambiante on dilue avec de l'eau et on extrait avec de l'éther. On sèche la phase organique sur sulfate de sodium puis évapore le solvant à siccité.

Par distillation du résidu on obtient un liquide jaune (9,36 g); E./0,6 mm Hg : 132—134°C.

b) Diisopropylamino-2 éthyl)-2 (pyridyl-2)-2 méthyl-4 pentène-4 nitrile.

Dans un tricol sous atmosphère d'azote on place 1.5 g d'hydrure de sodium (dispersion à 55—60% dans l'huile) et 60 ml de diméthylformamide (DMF). On ajoute goutte à goutte à température ambiante, 7,35 g du nitrile précédent dissous dans 30 ml de DMF. On agite pendant 30 minutes à température ambiante puis on ajoute 3 g de méthyl-2 chloro-3 propène dissous dans 30 ml de DMF. On agite 1 heure à température ambiante puis on évapore le DMF sous pression réduite. Le résidu est repris par de l'eau puis extrait avec de l'éther. On sèche la phase organique sur sulfate de sodium puis évapore à siccité.

On obtient un liquide orangé (10,2 g) que l'on utilise tel quel pour l'opération suivante.

c) CM 40348

On chauffe à reflux pendant 86 heures, 10,2 g du composé obtenu précédemment, 36 g de potasse, 150 ml d'éthanol à 95%, et 200 ml d'eau. On évapore l'alcool et extrait avec de l'écétate d'éthyle. On sèche la phase organique sur sulfate de sodium et évapore le solvant à siccité. On chromatographie le résidu sur une colonne d'alumine en éluant d'abord avec un mélange pentane-acétate d'éthyle puis avec de l'acétate d'éthyle. On obtient 4,73 g de solide blanc que l'on recristallise dans l'hexane, F : 92—93°C.

Exemples 3 à 22

En opérant selon la méthode A, mais en faisant varier le pyridyl acétonitrile de départ et/ou le réactif carbonylé $R_4COR_5$ utilisé dans la première étape et/ou la chaîne halogénée utilisée dans la troisième étape, on obtient les composés I réunis dans le tableau I ci-après.

Les produits de l'invention ont été étudiés en phamacologie animale et humaine et en particulier en vue de mettre en évidence leurs propriétés antiarythmiques et antiplaquettaires.

TABLEAU I

| Numéro de code | Position de substitution sur la pyridine | $R_1$ | n | 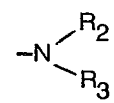 | Point de fusion, °C (solvant de cristallisation) |
|---|---|---|---|---|---|
| 7526 | 2 | 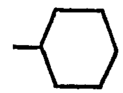 | 2 | 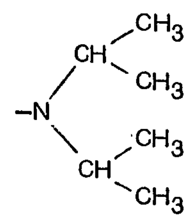 | 104–105 (éther isopropylique) |
| 7641 | 2 | 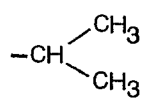 | 2 | ,, ,, | 85–86 (éther isopropylique) |
| 7827 | 2 | 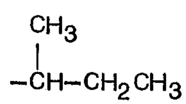 | 2 | ,, ,, | 90–91 (éther isopropylique) |
| 7828 | 2 | $-CH_2CH_3$ | 2 | ,, ,, | 93–94 (éther isopropylique) |
| 7855 | 2 | 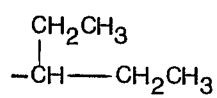 | 2 | ,, ,, | 62–63 (éthanol à 96°) |
| 7927 | 2 | $-CH_2CH_2CH_3$ | 2 | ,, ,, | 87–88 (éther isopropylique) |
| 7973 | 2 | $-CH_2CH_2CH_2CH_2CH_3$ | 2 | ,, ,, | 90–91 (éther isopropylique) |

TABLEAU I (suite 1)

| Numéro de code | Position de substitution sur la pyridine | $R_1$ | n | 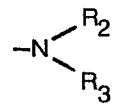 | Point de fusion, °C (solvant de cristallisation) |
|---|---|---|---|---|---|
| 7956 | 2 | 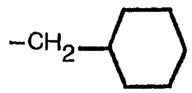 | 2 | 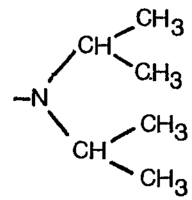 | 117 (éther isopropylique) |
| 7974 | 2 | 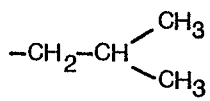 | 2 | 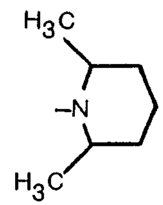 | 119–120 (éther isopropylique) |
| 7975 | 2 | ,, ,, | 2 | 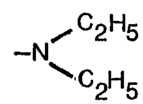 | 78–79 (hexane) |
| 7976 | 2 | 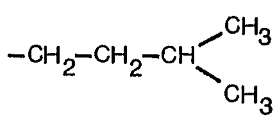 | 2 | 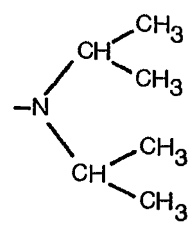 | 108–109 (éther isopropylique) |

TABLEAU I (suit 2)

| Numéro de code | Position de substitution sur la pyridine | $R_1$ | n | 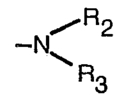 | Point de fusion, °C (solvant de cristallisation) |
|---|---|---|---|---|---|
| 40003 | 2 | 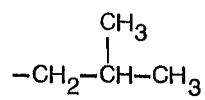 | 2 | 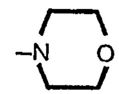 | 89–90 (éther isopropylique) |
| 40023 | 2 | 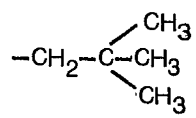 | 2 | 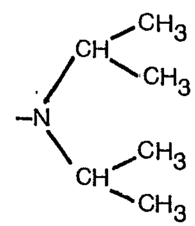 | 103–104 (éther isopropylique) |
| 40156 | 2 | 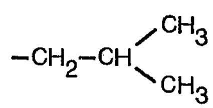 | 2 | 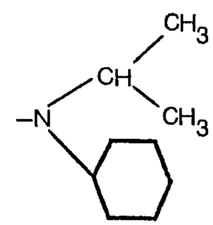 | 106–107 (éther isopropylique) |
| 40163 | 2 | 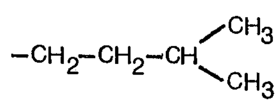 | 2 | 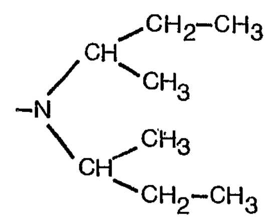 | 92–93 (éther isopropylique) |

TABLEAU I (suit 3)

| Numéro de code | Position de substitution sur la pyridine | $R_1$ | n | 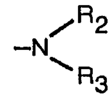 | Point de fusion, °C (solvant de cristallisation) |
|---|---|---|---|---|---|
| 40165 | 2 | 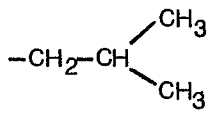 | 2 | 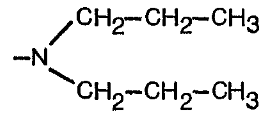 | 74–76 (éther isopropylique) |
| 40196 | 2 | 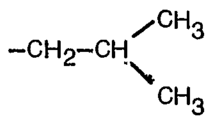 | 3 | 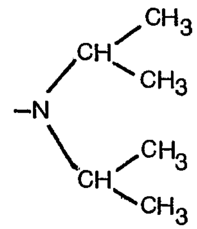 | 54–56 |
| 40226 | 2 | 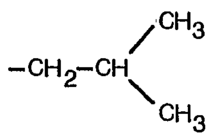 | 2 | 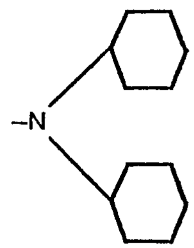 | 139–140 (éther isopropylique) |
| 40453 | 4 | 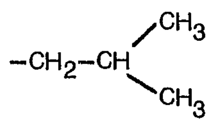 | 2 | 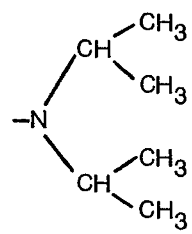 | 127–128 (éther isopropylique) |

TABLEAU I (suit 4)

| Numéro de code | Position de substitution sur la pyridine | $R_1$ | n | 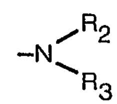 | Point de fusion, °C (solvant de cristallisation) |
|---|---|---|---|---|---|
| 40455 | 3 | 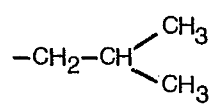 | 2 | 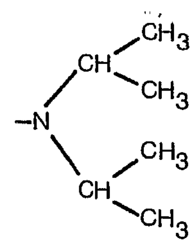 | 110–111 (éther isopropylique) |

*Mise en évidence des propriétés antiarythmiques.*

Protocole:

Le pouvoir antiarythmique de ces molécules a été apprécié sur un modèle animal d'arythmie ventriculaire.

Des chiens bâtards sont anesthésiés puis soumis à la mise en place, par cathétérisme rétrograde, d'une spire métallique dans le lit coronarien. Dans le même temps un micro émetteur modulateur de fréquence est fixé au dos de l'animal et relié à deux électrodes précordiales.

L'animal remis dans son box accuse alors une thrombose progressive de l'artère interventriculaire antérieure. Ainsi se constitue un infarctus myocardique localisé et transmural, générateur d'une activité électrique anormale mais répétitive : tachycardie ventriculaire.

Dans cet état les drogues sont administrées per os (P.O.) et le système télémétré permet de suivre en temps réel l'évolution de la disrythmie.

Un comptage des complexes systoliques sinusaux et pathologiques est assuré en permanence par des procédés électroniques. Ainsi peut-on quantifier la qualité et la durée d'action du produit.

Résultats:

Les résultats concernant divers produits sont rassemblés dans le tableau II ci-après.

L'activité des produits essayés sur la tachycardie ventriculaire est exprimée soit par le rétablissement du rythme sinusal, soit par une amélioration notable du rapport:

$$\frac{\text{nombre de complexes anormaux}}{\text{nombre de complexes sinusaux}}$$

TABLEAU II

| Produits n° | Dose mg/kg P.O. | Nombre d'animaux | Effet sur la tachycardie ventriculaire |
|---|---|---|---|
| CM 7526 | 50 | 3 | Rythme sinusal ou amélioration à 90% de 3 h 15 min à plus de 4 h 30 min |
| CM 7641 | 50 | 2 | Rythme sinusal ou amélioration entre 70—90% de 1 h 45 min à 6 h |
| CM 7827 | 50 | 1 | Amélioration à 75% durant 4 h |
| CM 7828 | 50 | 2 | Amélioration entre 60 et 95% de 2 h 30 min à 5 h |
| CM 7855 | 50 | 3 | Rythme sinusal ou amélioration à 80% de 30 min à 3 h |
| CM 7857 | 50 | 3 | Rythme sinusal ou amélioration à 85% de 3 h à plus de 4 h 30 min |
| CM 40023 | 50 | 5 | Amélioration entre 50 et 100% de 1 h à 2 h 45 min |
| CM 40156 | 50 | 2 | Amélioration à 50% de 1 à 2 h |

Chez l'homme, l'administration par voie orale d'une dose unique de 50 mg de CM 7857 conduit à une restauration du rythme sinusal.

*Mise en évidence des propriétés antiplaquettaires.*

Protocole:

Le pouvoir antiagrégant a été apprécié in vitro et exvivo selon la technique turbidimétrique de Born.

Les essais in vitro ont été réalisés sur plasma riche en plaquettes d'origine humaine. Le produit à tester est dissous extemporanément dans une solution isotonique de chlorure de sodium. Le produit est incubé à 37°C pendant 5 minutes en présence de plasma riche en plaquettes avant l'addition de l'argent agrégant.

Les essais ex-vivo ont été réalisés chez le babouin soumis à la diète hydrique la veille de l'essai. Le produit à tester est administré par voie buccale à une dose de 50 mg/kg. Les prélèvements sanguins pour l'analyse de l'agrégation plaquettaire sont effectués avant l'administration du produit, puis 1, 2, 3, 4 et 24 h après l'administration.

Les résultats sont exprimés en pourcent d'inhibition d'agrégation plaquettaire calculés par rapport aux contrôles (100% d'agrégation).

Résultats:

Les essais in vitro réalisés sur plasma riche en plaquettes humain ont montré que le CM 7857 pouvait antagoniser l'agrégation plaquettaire induite par le collagène. La concentration nécessaire pour inhiber de 50% l'agrégation plaquettaire est voisine de 80 $\mu$m.

Les essais ex-vivo ont été réalisés aprés administration orale d'une dose unique de 50 mg/kg chez quatre babouins. Pour cette dose, on observe une inhibition de 30% vis-à-vis de l'agrégation plaquettaire induite par l'ADP.

Chez l'homme l'administration par voie orale d'une dose unique de 50 mg de CM 7857 a permis la disparition des troubles de l'agrégation plaquettaire.

Ces résultats montrent que les produits selon l'invention sont doués d'une forte activité sur la dysrythmie. Ils présentent également une activité antiplaquettaire importante.

Par suite, les produits (I) peuvent étre utilisés en thérapeutique humaine comme protecteur du myocarde pour la correction des troubles du rythme ventriculaire d'origine ischèmique, ainsi que des troubles de l'agrégration plaquettaire.

Les produits pourront être présentés sous les formes galéniques correspondant à la voie orale (comprimés, gélules ...) et à la voie parentérale (ampoules injectables).

La dose nécessaire à une activité antiplaquettaire ou à la restauration du rythme sinusal chez l'homme est comprise entre 50 et 150 mg par voie intraveineuse et entre 400 et 800 mg par voie orale, par jour, environ.

A titre d'exemple, on indique la préparation galénique suivante:

| <u>Comprimés</u> | |
|---|---|
| CM 7857 | 0,200 g |
| Cellulose microcristalline | 0,140 g |
| Lactose | 0,140 g |
| Stéarate de magnésium | 0,020 g |
| | 0,500 g |

**Revendications**

1. Nouveaux dérivés de la pyridine de formule

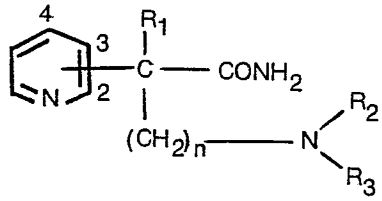

(I)

dans laquelle:

—le substituant de la pyridine est fixé en l'une des positions 2, 3 ou 4 du cycle pyridine,

—$R_1$ représente un alkyle droit ou ramifié saturé ou insaturé ayant 1 à 10 atomes de carbone un groupe cyclohexyle ou cyclohexyleméthyle,

—$R_2$ et $R_3$ représentent un groupe alkyle droit ou ramifié ayant 1 à 6 atomes de carbone ou un groupe cyclohexyle, ou encore les deux radicaux $R_2$ et $R_3$ constituent, avec l'atome d'azote auquel ils sont liés, un hétérocycle choisi parmi le groupe morpholino et le dimethyl-2,6 pipéridino.

—n étant égal à 2, 3 ou 4,

ainsi que les sels desdits dérivés avec des acides minéraux ou organiques, notamment des acides pharamaceutiquement acceptables.

2. Procédé de préparation des dérivés de la pyridine selon la revendication 1, dérivés dans lesquels le radical $R_1$ est un radical saturé, caractérisé en ce que l'on effectue les opérations successives suivantes:

a) on traite par un aldéhyde ou une cétone de formule $R_4$—$COR_5$, dans laquelle $R_4$ est H ou alkyle et $R_5$ est alkyle, un pyridylacétonitrile de formule:

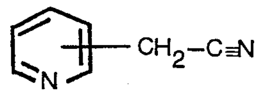

la réaction étant effectuée en présence d'acide acétique et de pipéridine, en milieu solvant,

b) on réduit catalytiquement le nitrile éthylénique obtenu dans la réaction précédente de façon à obtenir un nitrile saturé, ladite réduction étant réalisée de façon connue,

c) on soumet le nitrile saturé obtenu à une réaction d'alkylation par réaction avec un composé de formule:

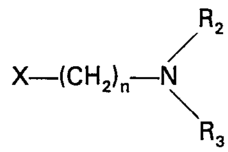

X étant un halogène,

d) on réalise ensuite l'hydrolyse de la fonction nitrile du produit obtenu dans la réaction précédente, cette hydrolyse étant effectuée de façon connue en soi, pour l'obtention de l'amide correspondant.

3. Procédé de préparation de dérivés de la pyridine selon la revendication 1, dérivés dans lesquels le radical $R_1$ est un radical alkyle insaturé, caractérisé en ce que l'on effectue les opérations suivantes:

a) on soumet un pyridylacétonitrile de formule:

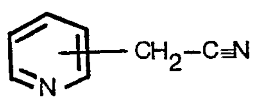

à une réaction d'alkylation avec un composé de formule

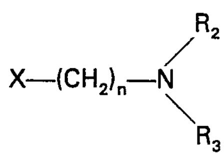

X étant un halogène,

b) on soumet le produit obtenu à une deuxième réaction d'alkylation à l'aide d'un composé de formule $R_1X$ dans lequel $R_1$ est un radical alkyle insaturé,

c) on réalise ensuite, de façon connue, l'hydrolyse de la fonction nitrile du produit obtenu de façon à conduire à l'amide correspondant.

4. Médicaments, utiles notamment pour leur activité antiplaquettaire et antiarythmique, caractérisés en ce qu'ils contiennent comme produit actif un dérivé selon la revendication 1.

5. Médicaments selon la revendication 4, caractérisés en ce qu'ils sont conditionnés en vue d'une administration orale ou parentérale.

6. Médicaments selon la revendication 5, caractérisés en ce qu'ils sont conditionnés pour une administration orale à une dose, chez l'homme, comprise entre environ 400 et environ 800 mg de substance active.

7. Médicaments selon la revendication 5, caractérisés en ce qu'ils sont conditionnés pour une administration intraveineuse à une dose, chez l'homme, comprise entre environ 50 et environ 150 mg de substance active.

**Patentansprüche**

1. Neue Pyridinderivate der Formel

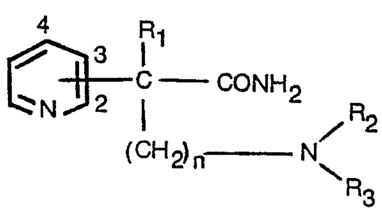

(I)

worin der Pyridinsubstituent an einer der Positionen 2, 3 oder 4 des Pyridinrings hängt, $R_1$ ein gerades

oder verzweigtes gesättigtes oder ungesättigtes Alkyl mit 1 bis 10 Kohlenstoffatomen, eine Cyclohexyloder Cyclohexylmethylgruppe darstellt, $R_2$ und $R_3$ für eine gerade oder verzweigte Alkylgruppe mit 1 bis 6 Kohlenstoffatomen oder eine Cyclohexylgruppe stehen, oder aber die beiden Radikale $R_2$ und $R_3$ mit dem Stickstoffatom, an das sie gebunden sind, einen Heterocyclus, ausgewählt aus der Gruppe Morpholino und Dimethyl-2,6-piperidino, bilden und n gleich 2, 3 oder 4 ist, sowie die Salze dieser Derivate mit Mineraloder organischen Säuren, insbesondere pharmazeutisch akzeptablen Säuren.

2. Verfahren zur Herstellung der Pyridinderivate nach Anspruch 1, worin das Radikal $R_1$ ein gesättigtes Radikal ist, dadurch gekennzeichnet, daß die folgenden Schritte ausgeführt werden;

a) Behandlung eines Pyridylacetonitrils der Formel

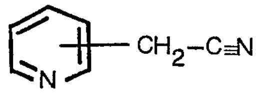

mit einem Aldehyd oder einem Keton der Formel $R_4$—$COR_5$, worin $R_4$ H oder Alkyl und $R_5$ Alkyl ist, wobei die Reaktion in Gegenwart von Essigsäure und Piperidin in einem Lösungsmittel durchgeführt wird,

b) katalytische Reduktion des bei der vorhergehenden Reaktion erhaltenen Äthylennitrils, sodaß ein gesättigtes Nitril erhalten wird, wobei die Reduktion auf bekannte Weise durchgeführt wird,

c) Alkylierung des erhaltenen gesättigten Nitrils durch Umsetzung mit einer Verbindung der Formel

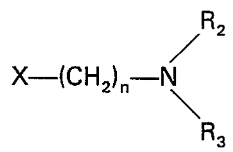

worin X ein Halogen ist,

d) danach Hydrolyse der Nitrilfunktion des bei der vorhergehenden Reaktion erhaltenen Produktes auf an sich bekannte Weise zur Erzielung des entsprechenden Amids.

3. Verfahren zur Herstellung von Pyridinderivaten nach Anspruch 1, worin das Radikal $R_1$ ein ungesättigtes Alkylradikal ist, dadurch gekennzeichnet, daß die folgenden Schritte ausgeführt werden:

a) Alkylierung eines Pyridylacetonitrils der Formel

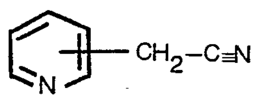

mit einer Verbindung der Formel

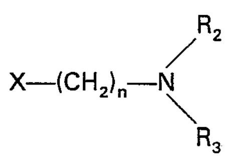

worin X ein Halogen ist

b) neuerliche Alkylierung des erhaltenen Produktes mittels einer Verbindung der Formel $R_1X$, worin $R_1$ ein ungesättigtes Alkylradikal ist,

c) danach Hydrolyse der Nitrilfunktion des erhaltenen Produktes auf bekannte Weise zur Bildung des entsprechenden Amids.

4. Medikamente, die insbesondere aufgrund ihrer aggregationshemmenden und antiarhythmischen Wirkung nützlich sind, dadurch gekennzeichnet, daß sie als Wirkstoff ein Derivat nach Anspruch 1 enthalten.

5. Medikamente nach Anspruch 4, dadurch gekennzeichnet, daß sie für orale oder parenterale Verabreichung aufbereitet sind.

6. Medikamente nach Anspruch 5, dadurch gekennzeichnet, daß sie zur oralen Verabreichung an Menschen zu einer Dosis zwischen etwa 400 und etwa 800 mg aktive Substanz aufbereitet sind.

7. Medikamente nach Anspruch 5, dadurch gekennzeichnet, daß sie für intravenöse Verabreichung an Menschen zu einer Dosis zwischen etwa 50 und etwa 150 mg aktive Substanz aufbereitet sind.

**Claims**

1. New pyridine derivatives of formula:

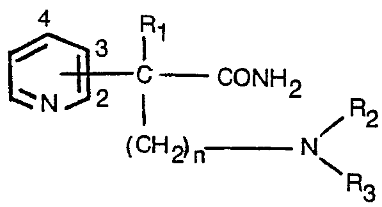

(I)

wherein:

—the pyridine substituent is fixed in one of the positions 2, 3 or 4 of the pyridine ring;

—$R_1$ is a straight or branched, saturated or non-saturated alkyl group with between 1 and 10 carbon atoms or a cyclohexyl or cyclohexyl methyl group

—$R_2$ and $R_3$ are a straight or branched alkyl group with between 1 and 6 carbon atoms or a cyclohexyl group or else the two groups $R_2$ and $R_3$ with the nitrogen atom to which they are bonded constitute a heterocycle selected from the morpholino and 2,6-dimethyl piperidino group;

—n being equal to 2, 3 or 4,

as well as the salts from said derivatives with mineral or organic acids, and in particular pharmaceutically acceptable acids.

2. Method for preparing the pyridine derivatives according to claim 1, in which derivatives the radical $R_1$ is a saturated group, characterized in that it consists in the following successive steps:

a) an acetonitrile pyridyl of formula:

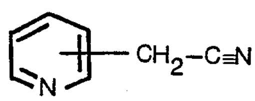

is treated with an aldehyde or a ketone of formula $R_4COR_5$, wherein $R_4$ is H or alkyl and $R_5$ is alkyl, the reaction taking place in the presence of acetic acid and piperidine in a solvent medium;

b) the ethylenic nitrile obtained from the preceding reaction is reduced catalytically so as to obtain a saturated nitrile, the said reduction being conducted in known manner;

c) The saturated nitrile obtained is subjected to an alkylation reaction with a compound of formula

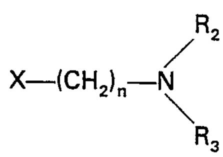

,

X being halogen;

d) then the nitrile function of the product of the above reaction is hydrolyzed in manner known per se, to obtain the corresponding amide.

3. Method for preparing the pyridine derivatives according to claim 1, in which derivatives the radical $R_1$ is a non-saturated alkyl radical, characterized in that it consists in the following operations:

a) subjecting an acetonitrile pyridyl of formula:

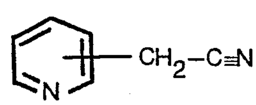

to an alkylation reaction with a compound of formula

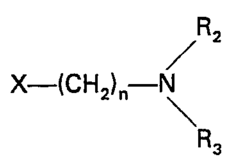

,

X being halogen.

b) subjecting the resulting product to a second alkylation reaction with a compound of formula $R_1X$ wherein $R_1$ is a non-saturated alkyl radical.

c) hydrolyzing, in known manner, the nitrile function of the product obtained so as to arrive at the corresponding amide.

4. Medicines, particularly useful for their activity as blood-platelets anti-aggregants and for their anti-arrhythmic activity, characterized in that they contain as active substance a derivative according to claim 1.

5. Medicines according to claim 4, characterized in that they are presented for oral or parenteral administration.

6. Medicines according to claim 5, characterized in that they are presented for oral administration at a dose for humans varying between about 400 and about 800 mg of active substance.

7. Medicines according to claim 5, characterized in that they are presented for intraveinous administration at a dose in humans varying between about 50 and about 150 mg of active substance.